# EUROPEAN PATENT APPLICATION

(11) **EP 1 992 346 A1**
(43) Date of publication of application: **19.11.2008**
(21) Application number: 06783798.9
(22) Date of filing: 12.07.2006
(51) Int. Cl.: A61K 31/4365, A61K 31/045

(54) **PHARMACEUTICAL COMPOSITIONS CONTAINING COMBINED PLATELET AGGREGATION INHIBITING SUBSTANCES FOR USE IN THE TREATMENT AND PREVENTION OF ISCHEMIC VASCULAR EVENTS**

(30) Priority: 20.12.2005 ES 200514086
(71) Applicant: WORLD-TRADE IMPORT-EXPORT, WTIE, A.G., 6320 Zug (CH)
(72) Inventor: GARCIA ARMENTA, María, Elena, C.P. 45020 Guadalajara, Jalisco (MX); ÁLVAREZ OCHOA, Víctor, Guillermo, C.P. 45222 Zapopan, Jalisco (MX); SANTOS MURILLO, Josefina, C.P. 44600 Zapopan, Jalisco (MX); GARCÍA ARMENTA, Patricia, C.P. 45222 Zapopan, Jalisco (MX)
(74) Representative: Carvajal y Urquijo, Isabel
(86) International application number: PCT/MX2006/000068
(87) International publication number: WO 2007/073131

(57) **Abstract**

The invention relates to the pharmaceutical industry in general and in particular to the pharmaceutical industry involved in the production of medicaments for controlling and preventing ischemic vascular events. More specifically, the invention relates to a composition comprising a combination of platelet aggregation inhibiting substances. The composition is **characterised in that** the aforementioned combination or association consists of a selected platelet aggregation inhibiting substance known as Clopidogrel and a platelet aggregation inhibiting substance having hypolipidaemic activity, known as Policosanol, in order to produce a produce which has a synergistic effect for the prevention and treatment of ischemic vascular events.

## Description

### FIELD OF THE INVENTION

The present invention relates to overall pharmaceutical industry and to pharmaceutical industry preparer of drugs for controlling cardiovascular events. More specifically, the invention relates to a composition formed by inhibitors of platelet aggregation such as an inhibitor of platelet receptors IIb/IIIa, such as Clopidogrel, and a natural mixture of aliphatic alcohols, such as Policosanol, for pharmaceutical use of the composition for prevention of ischemic vascular events; the composition produces a combined product with improved properties and a synergistic effect.

### BACKGROUND OF THE INVENTION

The major cause of myocardial ischemia is atherosclerosis of the epicardial coronary arteries. The reduction of these vessels causes an absolute decrease of myocardium perfusion in the basal state, or restricts the proper increase of perfusion when the flow requirement increases.

The ischemic heart disease (IHD) is the most reliable index currently existing to diagnose atherosclerosis, and is the major cause of mortality in males older than 35 years old and in all people older than 45 years old. It is most frequently presented in white race males than en females thereof. The frequency of its mention in death certificates is 4.5-fold higher as a conditioning cause than as a basic cause.

In 2003, 50 thousand cases of ischemic heart diseases were reported in Mexico, being the second cause of death in that country and the first in men older than 60 years old, the most common risk-factor for the development of cardiac conditions are dyslipidemia, diabetes mellitus and hypertension.

The epicardial coronary arteries are one of the main locations of atherosclerotic disease. It is believed that the main risk-factors of atherosclerosis (elevation of plasmatic LDLs, lowering of HDLs, smoke cigarettes, diabetes mellitus and hypertension) alter the normal functions of vascular endothelium. The dysfunction of vascular endothelium and abnormal interaction with blood monocytes and platelets leads to the accumulation, under the intima, of abnormal cells and waste (i.e. atherosclerotic plaques) which develop in non-regular manner at different segments of the epicardial tree and which ultimately produce segmental reductions of its section.

### Formation of blood platelet thrombus

The formation of blood platelet thrombus performs a fundamental role in the pathogenesis of acute coronary symptoms (ACS), including unstable angina (UA), non Q-wave myocardial infarction (NQMI) and Q-wave myocardial infarction and ST-elevation.

Processes of platelet adhesion, activation and aggregation take part in the blood platelet thrombus formation. When endothelial damage is produced, the adhesion of circulating platelets to ligands of damaged vascular wall begin, such as the exposed collagen, vitronectin, von Willebrand factor and thrombin. The adhesion is followed by the platelet activation, through multiple mechanisms involving several agonists, including collagen, thrombin, adenosine diphosphate (ADP), thromboxane A2, serotonin, noradrenalin and adrenalin, many of which are found in high concentrations at the site of vascular lesion.

Because of activation, the platelet degranulation is produced, releasing more ADP, thromboxane A2 and generation of more thrombin from the circulating prothrombin. All of which increases the extent of platelet activation, and recruits a higher number of platelets to the thrombus scenario.

Platelet activation induces a conformational change on platelet, which carries the exposure and activation of the platelet receptors IIb/IIIa of GP, stored within the platelet, to the surface thereof. Once activated, the receptor IIb/IIIa binds to fibrinogen, which is its primary ligand; the platelets aggregate and form a real thrombus.

The conversion of fibrinogen into fibrin by the thrombin will finally stabilize the thrombus. The binding of fibrinogen to receptor IIb/IIIa is the only mediator of platelet aggregation and is independent from platelet activation mechanisms. Therefore, the binding of the fibrinogen to receptor IIb/IIIa has been denoted the common final via in blood platelet thrombus formation.

The receptor of GP, IIb/IIIa, belongs to the family of integrins. It is the most abundant protein on the platelet surface, with 50,000 to 80,000 copies; another reserve maintains in the intracellular stocks. It is composed by two protein units: subunit a (IIb), made up of an extracellular heavy chain and of another light chain with 3 segments located in cytoplasm, cellular membrane and extracellular level, respectively; and by subunit b (IIIa), made up of a single chain with an intracytoplasmic tail, a transmembranal segment and another located outside the cell.

Being the instability of atherosclerotic plaque complicated with rupture and thrombosis the main element to be controlled; for which there are several medicaments of proven efficiency in acute phase, among the principal ones it is found: anticoagulants and inhibitors of platelet aggregation.

As mentioned above, among the medicaments used as inhibitors of platelet aggregation, it is of interest to revise the inhibitors of platelet receptors IIb/IIIa, and a natural mixture of aliphatic alcohols such as Policosanol which besides having an activity as a platelet aggregation inhibitor, it has also been widely used as an hypocholesterolemic agent.

### INHIBITORS OF PLATELET RECEPTORS IIb/IIIa

Inhibitors of receptors IIb/IIIa are potent blockers of the receptor that mediates the platelet aggregation and thus block the common final path of blood platelet thrombus formation.

### CLOPIDOGREL

Clopidogrel is inactive in vitro and requires hepatic activation in order to exert its effects as a platelet aggregation inhibitor. The active metabolite inhibits selectively and irreversibly the ADP-induced platelet aggregation, preventing the binding of adenosine diphosphate to platelet receptor. In this manner, the activation of the glycoproteic complex GIIb/IIIa results altered. Because this complex is the most important receptor for fibrinogen, its inactivation prevents fibrinogen from binding to the platelets, which ultimately inhibits platelet aggregation.

Because the active metabolite of Clopidogrel irreversibly modifies the platelet receptor, the platelets exposed to the drug remain altered the rest of their life. The active metabolite of Clopidogrel also inhibits platelet aggregation induced by other agonists. However, it does not inhibit phosphodiesterase.

Clopidogrel is orally administered. Is inactive in vitro and needs a biotransformation which is performed in liver in order to achieve its activation. This activation is believed to be performed by the isoenzyme system pertaining to subfamily CYP 1A of cytochrome P450. The active compound being quite labile and it has not been identified, thus the pharmacokinetic profile corresponds to that of an inactive primary metabolite, i.e. a carboxylic acid representing about 85% of metabolites circulating in the plasma.

The absorption of Clopidogrel is of 50% and it is not significantly affected by food. The maximum plasmatic concentrations of primary metabolite are achieved one hour after a dose of 75 mg. Two hours after an oral dose, plasmatic concentrations of the unaltered drug are no longer detectable. Clopidogrel and the main metabolite thereof are reversible bound to plasmatic proteins (98 and 94%, respectively). About 50% of Clopidogrel radioactively marked is eliminated through urine while 46% is eliminated though feces within a five-day period; the plasmatic half-life is about 8 hours.

Two hours after a single oral dose, it is already observed an inhibition of platelet aggregation. With iterative doses of 75 mg per day, the maximum inhibition of platelet aggregation is achieved within 3-7 days. In balanced conditions, platelet aggregation is inhibited in a 40-60%. The extension of bleeding-time is not significantly affected by age, renal dysfunction or gender. Platelet aggregation as well as bleeding-time gradually become to their basal level within five days after the treatment discontinuation.

### POLICOSANOL

Policosanol is a product made up of a natural mixture of higher primary aliphatic alcohols isolated from sugar cane wax. It is a medicament that acts by inhibiting the cholesterol synthesis.

Its main effect is to reduce the total cholesterol concentration, fundamentally at the expense of LDL. It also moderately increases HDL concentration, while its effect on triglycerides is discrete. Because of this, its indication as a hypocholesterolemic agent is for patients with primary hyperlipoproteinemia of the type II a (elevation of cholesterol values and LDL-C) or type II b (elevation of cholesterol values, LDL-C, and serum triglycerides). However, it is always desirable to remember that the diet constitutes the basis of lipid reducer treatment. By this reason, Policosanol should be indicated in patients who do not decrease their LDL-C and cholesterol amounts with diet, and such diet should be maintained during medicament treatment.

Studies on hypercholesterolemia associated to diabetes mellitus support the criterion that treatment with Policosanol do not interferes with glycemic control.

Apart from the effect on lipid metabolism, Policosanol has demonstrated that reduce thromboxane concentration associated with a decrease of platelet aggregation. This platelet aggregation inhibiting action has been tested in healthy volunteers.

Policosanol low toxicity and effectiveness has been tested in various preclinical and clinical studies.

In short, the formation of blood platelet thrombus performs a fundamental role in the pathogenesis of acute coronary syndromes (ACS), including unstable angina (UA), non Q-wave myocardial infarction (NQMI) and Q-wave myocardial infarction and ST-elevation. The instability of atherosclerotic plaque is the main element to be controlled.

Currently, a great majority of patients use combined medicaments in order to minimize ischemic vascular disease; however, the demographic characteristics of population, type of ingested carbohydrate-rich food, daily activities, and passivity are silent risk-factors which contribute nowadays to the appearance of ischemic vascular events in individuals younger than 60 years old.

Due to this reason, tests were performed with such combination in order to evaluate the synergy of both compounds as inhibitors of platelet aggregation, as well as the hypocholesterolemic effect, using fewer doses of ingredients.

### BRIEF DESCRIPTION OF THE INVENTION

The present invention realize in compositions comprising platelet aggregation inhibiting substances, such as an inhibitor of platelet receptors IIb/IIIa, such as Clopidogrel, and a natural mixture of aliphatic alcohols, such as Policosanol, for pharmacological use of the composition in the prevention of ischemic vascular events; the composition produces a combined product with improved properties and a synergistic effect.

In one of the preferred embodiments, the composition is characterized in that the medicament requiring a fewer dose is Policosanol. In other of the embodiments also preferred, Policosanol is present at a concentration less than 20 mg Policosanol per dosage unit. It could also be able to confirm, from several tests, that Policosanol could also be present at a concentration of 10 mg per dosage unit, providing good results.

Concerning Clopidogrel, in one of the embodiments, is present as Clopidogrel bisulphate at a concentration of 75 mg per dosage unit.

In other of the embodiments of the present invention, the use of the compositions above mentioned was determined for treatment and prevention of ischemic vascular events.

### DETAILED DESCRIPTION OF THE INVENTION

According to the present invention, a pharmacological composition was made comprising a combination of an inhibitor of platelet receptors IIb/IIIa known as Clopidogrel and a natural mixture of aliphatic alcohols known as Policosanol, which combination produces a synergistic effect as a platelet aggregation inhibitor with fewer doses of ingredients, as well as a hypocholesterolemic effect, being better tolerated and with less adverse effects in preventing ischemic vascular events.

### EXAMPLE 1:

A comparative CLOPOL (Clopidogrel vs. Policosanol) study was made for evaluating the effectiveness and reliability of an inhibitor of platelet receptors IIb/IIIa, such as Clopidogrel alone, vs. a natural mixture of aliphatic alcohols, such as Policosanol alone, in order to evaluate its effect as platelet aggregation inhibitor and test its effectiveness when used in combination (Clopidogrel/Policosanol). It was a double blind, 10-day duration study that was performed in 20 healthy volunteers with an age ranging from 26 to 40 years old. The platelet activation was measured by changes in light transmission of plasma rich in platelets stimulated by a synthetic analogue of TXA₂, U46619.

### Results

U46619 stimulated platelet aggregation, being significantly inhibited by Clopidogrel in a dependent-dose manner as compared to Policosanol alone; however, when used in combination, Clopidogrel/Policosanol produced synergy, reducing the U46619-induced platelet aggregation in less time and with absence of collateral effects.

### Conclusions

The results of this study contribute new evidences, which demonstrates aggregation inhibiting effects with the Clopidogrel/Policosanol combination, producing a synergistic effect and decreasing the possibility of collateral effects.

### EXAMPLE 2:

A comparative CLOPOL-I (Clopidogrel vs. Policosanol) study was made for evaluating effectiveness and tolerance of a combination comprised by an inhibitor of platelet receptors IIb/IIIa, Clopidogrel, with a natural mixture of aliphatic alcohols, such as Policosanol, as compared to the combination of an inhibitor of platelet receptors, such as Clopidogrel, and a platelet aggregation inhibitor, such as acetyl salicylic acid. It was a double blind, 4-week duration study which included 100 patients with risk factors for ischemic vascular events, the inclusion criteria were: people older than 40 years old, both genders, positive tobacco smoking, moderated obesity IMC32-34.9 kg/m², total cholesterol < 200, passivity and antecedents of gastrointestinal disorders (acid-peptic disease). The patients were randomized to receive: Group 1 (n=50) the Clopidogrel/Policosanol combination once a day, Group 2 (n=50) Clopidogrel/acetyl salicylic acid once a day.

### Results

Demographic data included: average age 45.6 years, tobacco smoking in 96% of individuals, average IMC of 33.1 kg/m², average total cholesterol 234, passivity in 96% and antecedents of acid-peptic disease in 100% of the enlisted individuals. From patients who received the Clopidogrel/Policosanol combination (n=50), 100% concluded the study, only in 4% of individuals, adverse events were documented characterized by: semiliquid evacuations, which did deserve neither to suspend the study therapy nor to add concomitant therapy. In Group 2 which received Clopidogrel/acetyl salicylic acid combination, only 60% concluded the research study, being reported adverse events in 40%, characterized by abdominal pain (12%), nausea (16%) and melena (12%).

The group that received the Clopidogrel/Policosanol combination had a reduction percentage in serum-cholesterol levels of (35%) relative to the basal visit.

**Conclusions;** In patients with antecedents of acid-peptic disease, which deserve preventive therapy with inhibitors of platelet aggregation, the combination of Clopidogrel/Policosanol is an excellent therapeutic alternative.
□ Synergistic effect as a platelet aggregation inhibitor.
□ Significantly reduces the death risk for cardiovascular, myocardial infarction, ictus and refractory ischemia.
□ Significantly reduces serum-cholesterol levels (involved in genesis of atherosclerotic plaque).
□ Benefits of Clopidogrel/Policosanol as a preventive therapy in ischemic vascular disease.
□ Benefits of minimum adverse events reported, with excellent tolerance.
□ Single combination of inhibitors of platelet aggregation and a hypocholesterolemic agent indicated in individuals with acid-peptic disease or in those where concurrent administration of acetyl salicylic acid is contraindicated.

### EXAMPLE 3.

A comparative CLOPOL-II study was made for evaluating the effectiveness and tolerance of a combination comprised by an inhibitor of platelet receptors IIb/IIIa, Clopidogrel, with a natural mixture of aliphatic alcohols, such as Policosanol, comparing the combination of an inhibitor of platelet receptors, such as Clopidogrel, and a platelet aggregation inhibitor, such as acetyl salicylic acid, in individuals with coronary artery disease.

It was a double blind, 12-week duration study, randomized to two groups of treatment. Group 1 to receive the Clopidogrel/Policosanol combination. Group 2 Clopidogrel/acetyl salicylic acid, 20 individuals were subjected to study with antecedents of coronary artery disease (CAD): myocardial infarction, stable or unstable angina, all those candidates to receive platelet aggregation inhibiting treatment, and who have not received any drug of this type 7 days before their admission to the study. The diagnosis of ischemic heart disease was supported in the clinical and electrocardiographic chart, as well as by coronary arteriography; each individual was asked a written consent in order to become part of the study.

### Results

The demographic data for the population in study were: average age 63.4 years; in order to evaluate the action of Clopidogrel/Policosanol vs. Clopidogrel acetyl salicylic acid, the following tests were practiced before starting the study therapy (basal sample) and in each of the follow-up visits, test of ADP- and collagen-induced platelet aggregation, bleeding time (normal from 3 to 7 minutes) and the concentration of plasmatic fibrinogen was determined.

Were included 20 individuals with coronary artery disease, from which 16 concluded the study; the demographic characteristic was: male sex (80%). The disease evolution time was 3.4 years, the personal pathologic antecedents reported with higher frequency were: type II diabetes mellitus (45%), hyperlipidemia (95%) and essential arterial hypertension (85%).

A progressive decrease of ADP-induced platelet aggregation during 12 weeks under observation was noted. The aggregation was from 90.7% to 54.6% in treatment group 1, in the group 2 was from 91.3% to 55.3%. The percentage of platelet aggregation reduction from administration of the study therapy confirms a considerable decrease in both treatment groups, which was similar for both treatment groups.

The bleeding times were significantly extended from 4.1 to 15.4 in group 2, as compared to group 1, from 4.3 to 14.6 minutes at 6th and 12th week, respectively, which represented an increase from 2.8 to 2.5 times as for basal value. When determining the plasmatic fibrinogen, no significant changes were observed during treatment.

In total, 4 individuals (20%) had to be retired before the end of the study. The individuals corresponded to Group 2 (Clopidogrel/acetyl salicylic acid). The events that motivated the suspension were nausea, gastric acidity, and abdominal pain.

**Conclusions:** It can be concluded that the Clopidogrel/Policosanol combination produces a significant reduction of ADP-dependent platelet aggregation similar to Clopidogrel/acetyl salicylic acid in individuals with coronary artery disease; however, the absence of adverse events of Clopidogrel/Policosanol demonstrated an excellent safe profile.

## Claims

1. Synergistic pharmaceutical composition comprising a synergistic composition of an inhibitor of platelet receptors IIb/IIIa, and a natural mixture of aliphatic alcohols for the prevention of ischemic vascular events.

2. Pharmaceutical composition according to claim 1 comprising the synergistic combination, wherein the inhibitor of platelet receptors IIb/IIIa is Clopidogrel and/or any of salts thereof, and a natural mixture of aliphatic alcohols such as Policosanol and/or any of its salts.

3. A pharmaceutical composition according to claims 2, 3, and 4, wherein in the medicament requiring a fewer dose is Policosanol.

4. A pharmaceutical composition according to claim 2, wherein Clopidogrel is in the form of Clopidogrel bisulphate.

5. A pharmaceutical composition according to claim 2, wherein Clopidogrel is present in a concentration of 75 mg per dosage unit.

6. A pharmaceutical composition according to claim 2, wherein Policosanol is present in a concentration less than 20 mg of Policosanol per dosage unit.

7. A pharmaceutical composition according to claim 8, wherein Policosanol is present in a concentration of 10 mg per dosage unit.

8. Pharmaceutical compositions according to all previous claims, wherein acetyl salicylic acid, dipiridamol, ticlopidine, abciximab, eptifibatide, tirofiban, indobufen, triflusal or picotamide are excluded from formulation.

9. Pharmaceutical compositions according to all previous claims, wherein inhibitors of platelet receptors IIb/IIIa such as: xemilofiban, ordobofiban, sibrafiban, roxifiban, lotrafiban, lefradafiban are excluded from formulation.

10. Pharmaceutical compositions according to all previous claims, wherein substances such as inhibitors of platelet glycoprotein receptors IIb/IIIa, such as: abciximab, epifibatide, tirofiban are excluded from the formulation.

11. The use of the compositions according to claim 2 for the treatment and prevention of ischemic vascular events.
